Europäisches Patentamt

European Patent Office

Office européen des brevets

(11) Veröffentlichungsnummer: **0 360 729**
**A2**

# EUROPÄISCHE PATENTANMELDUNG

(21) Anmeldenummer: 89730196.6

(22) Anmeldetag: 18.09.89

(51) Int. Cl.⁵: **A 61 B 17/58**

(30) Priorität: 21.09.88 DE 8812077
02.01.89 DE 8900080

(43) Veröffentlichungstag der Anmeldung:
28.03.90 Patentblatt 90/13

(84) Benannte Vertragsstaaten:
AT BE CH DE ES FR GB GR IT LI NL SE

(71) Anmelder: MECRON MEDIZINISCHE PRODUKTE GMBH
Nunsdorfer Ring 23-29
D-1000 Berlin 48 (DE)

(72) Erfinder: Steür, Gerhard
Essener Strasse 8
D-1000 Berlin 21 (DE)

Sauer, Günther, Dr.-Ing.
Fregestrasse 72
D-1000 Berlin 41 (DE)

(74) Vertreter: Christiansen, Henning, Dipl.-Ing.
Patentanwalt CHRISTIANSEN Pacelliallee 43/45
D-1000 Berlin 33 (DE)

(54) Bausatz für die interne Fixation.

(57) Bausatz für die interne Fixation, insbesondere für die Fixation von Wirbelkörpern, aufweisend ein Links-und/oder ein Rechtsgewinde, wobei ein mit einem Außengewinde versehener Bereich am freien Ende des Gewindes nach wenigen Gewindegängen eine Zone aufweist, in der ein mit Titannitrid überzogener Bereich an einen nicht überzogenen Bereich grenzt (15, 16) und/oder ein eine Gewindeart aufweisendes Bauelement (3) bzw. ein entsprechender Teil (12) eines Bauelements (1) an einem relativ größeren Oberflächenbereich (14) mit einer Beschichtung versehen ist als ein die andere Gewindeart aufweisendes Bauelement (2) bzw. der entsprechende Teil (11) des Bauelements, wobei die restliche Oberfläche des aus Edelstahl oder Titan bestehenden Bauelements seine natürliche Silberfarbe aufweist.

Fig.1

EP 0 360 729 A2

**Beschreibung**

## Bausatz für die interne Fixation

Die Erfindung betrifft einen Bausatz für einen Fixateur intern der im Oberbegriff des Anspruchs 1 angegebenen Art.

Derartige Fixateure dienen vorrangig zur inneren Fixation bei Brust- und Lendenwirbelsäulendefekten.

Die Implantation herkömmlicher Fixateure erfordert aufgrund von deren Kleinheit ein hohes Maß an Geschicklichkeit und Erfahrung des implantierenden Chirurgen. Unter Operationsbedingungen ist insbesondere schlecht zu erkennen, ob es sich bei den Teilen des Bausatzes oder bei Gewindeansätzen dieser Teile jeweils um solche mit einem Links- oder solche mit einem Rechtsgewinde handelt. Die Unterscheidung zwischen einem rechtsdrehenden und einem linksdrehenden Gewindebereich ist daher oft nur durch versuchsweises Schrauben der Überwurfmutter möglich.

Um eine ausreichende Belastbarkeit des Fixateurs zu garantieren, ist ferner eine Mindesteinschraubtiefe der ein Außengewinde tragenden Bereiche unbedingt erforderlich. Diese läßt sich jedoch nicht abschätzen, da die Gesamtlänge der Außengewinde unterschiedlich ist. Bei der Einstellung eines derartigen Fixateurs am Patienten ist aber die Kenntnis der Mindesteinschraubtiefe unerläßlich, damit sich beim Einstellen nicht das mit einem Außengewinde versehene Element versehentlich von dem entsprechenden mit dem Innengewinde versehenen Element trennt und somit die Fixierung gefährdet ist.

Da die Oberfläche bei den bekannten Implantaten einheitlich bezüglich der optischen Reflexionseigenschaften ausgebildet ist, fehlen also Bereiche, die der Kennzeichnung dienen können.

Der Erfindung liegt die Aufgabe zugrunde, einen Fixateur der eingangs genannten Gattung anzugeben, der eine Beschichtung aufweist, die sich bezüglich ihrer optischen Eigenschaften, insbesondere bezüglich der Reflexionswirkung von nicht beschichteten Bereichen unterscheidet, aber trotzdem eine gute Biokompatibilität aufweist, wobei insbesondere auch die kontrastierende Beschichtung größerer Flächen möglich sein soll.

Diese Aufgabe ist mit den kennzeichnenden Merkmalen des Anspruchs 1 gelöst.

Überzüge aus Titannitrid sind bei Implantaten für die operative Knochenbehandlung grundsätzlich bekannt, wie beispielsweise aus der DE-PS 19 43 801.

Die Erfindung beruht auf der Erkenntnis, daß TiN-Überzüge infolge ihrer sich vom übrigen silberfarbigen Material unterscheidenden goldenden Farbe kontrastierend abheben, so daß damit verschiedene Funktionsbereiche von Bauelementen des Bausatzes in günstiger Weise, d.h. dauerhaft und körperkompatibel unterschieden werden können, wenn nämlich beschichtete Bereiche neben nicht beschichteten Bereichen vohanden sind und sich infolgedessen kontrastierend abheben. Dabei ist die erfindungsgemäße Beschichtung besonders günstig, da sie die kontrastierende Behandlung auch größerer Oberflächenbereich ermöglicht.

Da TiN-Schichten goldfarben erscheinen, heben sie sich auch unter Operationsbedingungen deutlich von dem Stahlgrau der aus Edelstahl- oder Titanlegierungen bestehenden übrigen Elemente des Bausatzes ab. Ein weiterer Vorteil des TiN-Überzuges besteht in seiner ausgezeichneten Biokompatibilität. Es hat sich gezeigt, daß TiN-beschichtete Edelstahlimplantate nach sechs Wochen Implantationszeit einen besseren Affinitätsindex als unbeschichtete Edelstahlimplantate aufweisen (Hayashi, K. et. al.; "The screening of metal implants coated with several types of ceramics"; The third biomaterial congress; April 21-25, 1988; Icyotot; Japan). Außerdem besitzen TiN-beschichtete Edelstahlimplantate eine höhere Korrossionsbeständigkeit als unbeschichtete Implantate.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 eine Seitenansicht eines Außengewinde aufweisenden Bauteils des erfindungsgemäßen Bausatzes für einen Fixateur sowie

Figuren 2 und 3 zwei an das erste Bauteil angepaßte Teile mit Innengewinde.

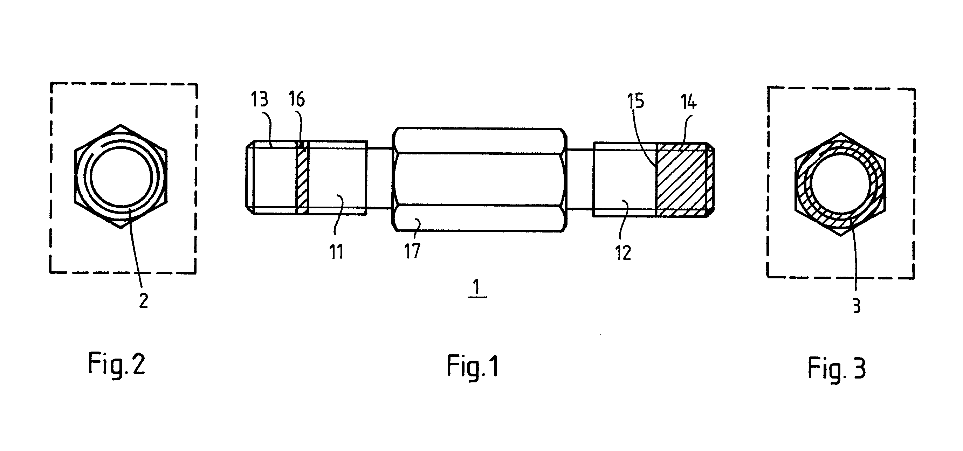

Bei dem in Figur 1 bis 3 dargestellten Ausführungsbeispiel eines Bausatzes ist ein mit Außenwinden versehenes Verstellelement 1 vorgesehen, welche in zwei in den Figuren 2 und 3 dargestellte Bauteile 2 und 3 mit Innengewinde eingreifen, die ihrerseits mittels in der Zeichnung nur schematisch gestrichelt dargestellter Ansätze, Eingriffsmittel oder Zusatzelemente mit den zu fixierenden Teilen des menschlichen Skeletts, insbesondere mit Wirbelkörpern oder Knochenfragmenten verbindbar sind.

Das Verstellelement 1 ist mit zwei Gewindestangen 11 und 12 versehen, welche jeweils ein Links- und ein Rechtsgewinde 13 bzw. 14 aufweisen. Bei der in der Zeichnung dargestellten Ausführung ist die linke Gewindestange mit einem Linksgewinde und die rechte Gewindestange mit einem Rechtsgewinde ausgestattet. Entsprechendes gilt für die zugeordneten Bauteile 2 und 3 gemäß Figuren 2 und 3.

Zwischen den Gewindestangen 11 und 12 ist ein Betätigungssechskant 17 angeordnet, der mittels eines Schraubenschlüssels verstellbar ist, so daß die beiden Gewindestangen 11 und 12 in die Innengewinde der Teile 2 und 3 einschraubbar sind. Durch eine Verstellung des Sechskants ist damit der Abstand der beiden Bauteile 2 und 3 relativ zueinander einstellbar.

Zweckmäßigerweise werden vor der Implantation des Fixateurs die beiden Schraubelemente 2 und 3 auf die jeweiligen Außengewinde 13 bzw. 14 des Verstellelements 1 bis zur Hälfte aufgeschraubt.

Die bei den aus Edelstahl bestehenden Bauelementen des Fixateurs 1 bis 3 mit Titannitrid überzogenen Bereiche sind in der Zeichnung schraffiert dargestellt. Der außenliegende Bereich 14 der Gewindestange ist mit TiN überzogen, wobei dieser Überzug unterhalb der Mitte des Gewindebereichs endet. Eine Grenze 15 zwischen mit Titannitrid überzogenem und nicht überzogenem Bereich verläuft tangential ringförmig um die Gewindestange herum. Der gegenüberliegende mit einem Linksgewinde versehene Teil (Gewindestange 11) ist mit einem Überzug von TiN in Form eines Rings 16 versehen.

Dieser Ring 16 verläuft in einem Abstand vom äußeren Ende der Gewindestange 11, der dem Abstand der Kante 15 des geschlossenen Überzugs 14 auf der Gewindestange 12 entspricht. Durch diese Art der Markierungen erkennt der Chirurg bei der Einstellung durch Erscheinen des goldfarbigen Bereiches ohne näheres Nachdenken zwangsläufig, daß er das Ausschrauben der Gewinde, d.h. das Betätigen des Sechskants 13 zum Entfernen der Teile 2 und 3 voneinander nicht weiter fortsetzen darf, da sonst die Gefahr besteht, daß die Gewindestange 11 bzw. 12 aus dem entsprechenden Gegenelement herausgerät.

Gleichzeitig unterscheiden sich die Überzüge an den Rechts- und Linksgewinden, wobei die Gewindestange 12 in ihrem äußeren Teil vollständig mit einer Schicht aus Titannitrid überzogen ist, was auch entsprechend für das Bauelement 3 im Hinblick auf seinen auf die Gewindestange 12 aufschraubbaren Bereich zutrifft (Schraffur in der Zeichnung). Der Chirurg wird auch hier ohne besondere Überlegung die einander zugeordneten Teile zusammenfügen, so daß sich der Montagevorgang des Fixateurs unter Operationsbedingungen beschleunigt und Fehlversuche von vorn herein ausgeschlossen sind.

Die beschichteten kontrastierenden Bereiche der Implantate unterscheiden sich bezüglich ihrer Oberflächen-Reflexionseigenschaften deutlich von nicht behandelten Bereichen und ermöglichen damit eine gute optische Unterscheidung.

Die Erfindung beschränkt sich in ihrer Ausführung nicht auf das vorstehend angegebene bevorzugte Ausführungsbei spiel. Vielmehr ist eine Anzahl von Varianten denkbar, welche von der dargestellten Lösung auch bei grundsätzlich anders gearteten Ausführungen Gebrauch machen.

## Patentansprüche

1. Bausatz für die interne Fixation, insbesondere für die Fixation von Wirkelkörpern, aufweisend ein Links-und/oder ein Rechtsgewinde, **dadurch gekennzeichnet,** daß ein mit einem Außengewinde versehener Bereich am freien Ende des Gewindes nach wenigen Gewindegängen eine Zone aufweist, in der ein mit Titannitrid überzogener Bereich an einen nicht überzogenen Bereich grenzt (15, 16) und/oder ein eine Gewindeart aufweisendes Bauelement (3) bzw. ein entsprechender Teil (12) eines Bauelements (1) an einem relativ größeren Oberflächenbereich (14) mit einer Beschichtung versehen ist als ein die andere Gewindeart aufweisendes Bauelement (2) bzw. der entsprechende Teil (11) des Bauelements, wobei die restliche Oberfläche des aus Edelstahl oder Titan bestehenden Bauelements seine natürliche Silberfarbe aufweist.

2. Bausatz nach Anspruch 1, **dadurch gekennzeichnet,** daß die Grenze (15) der Überzugsschicht aus TiN wenige Gewindegänge vom freien Ende des Gewindes (12) in tangentialer Richtung verläuft.

3. Bausatz nach einem der vorangehenden Ansprüche, **dadurch gekennzeichnet,** daß bei einem

angrenzend an einen Sechskant eine ein Rechtsgewinde aufweisende Gewindestange (12) und eine ein Linksgewinde aufweisende Gewindestange (11) tragenden Bauelement (1) eine der Gewindestangen (12) zumindest teilweise mit einer TiN-Schicht überzogen ist.

4. Fixateur nach einem der vorangehenden Ansprüche, **dadurch gekennzeichnet,** daß eine TiN-Schicht (16) ringförmig auf der Gewindestange (11) vorgesehen ist.

13  16                                    15  14

11        17                    12

1

Fig. 2                    Fig. 1                    Fig. 3

2                                              3

EP 0 360 729 A2